# EUROPEAN PATENT SPECIFICATION

(11) **EP 3 390 454 B1**
(45) Date of publication and mention of the grant of the patent: **04.02.2026**
(21) Application number: 16876653.3
(22) Date of filing: 15.12.2016
(51) Int. Cl.: A61K 39/395, A61P 35/02, C07K 16/28, C07K 16/46

(54) **A RECOMBINANT BI-SPECIFIC POLYPEPTIDE FOR COORDINATELY ACTIVATING TUMOR-REACTIVE T-CELLS AND NEUTRALIZING IMMUNE SUPPRESSION**
REKOMBINANTES BISPEZIFISCHES POLYPEPTID ZUR KOORDINIERTEN AKTIVIERUNG VON TUMORREAKTIVEN T-ZELLEN UND ZUR NEUTRALISIERUNG DER IMMUNSUPPRESSION
POLYPEPTIDE RECOMBINANT BISPÉCIFIQUE POUR L'ACTIVATION COORDONNÉE DE LYMPHOCYTES T RÉACTIFS À UNE TUMEUR ET LA NEUTRALISATION DE LA SUPPRESSION IMMUNITAIRE

(30) Priority: 17.12.2015 US 201562268682 P; 28.06.2016 US 201662355422 P
(43) Date of publication of application: 24.10.2018
(73) Proprietor: University of Maryland, Baltimore County, Baltimore, MD 21250 (US)
(72) Inventor: OSTRAND-ROSENBERG, Suzanne, Columbia, MD 21045 (US); CARTER, Darryl, L., White Marsh, MD 21162 (US)
(74) Representative: ABG Intellectual Property Law, S.L.
(86) International application number: PCT/US2016/066842
(87) International publication number: WO 2017/106453

(56) References cited:
- WO-A1-2013/079174
- WO-A1-2016/020065
- WO-A1-2016/034085
- WO-A2-2015/112534
- US-A1- 2015 024 489
- ROY S. HERBST ET AL: "Predictive correlates of response to the anti-PD-L1 antibody MPDL3280A in cancer patients", NATURE, vol. 515, no. 7528, 26 November 2014 (2014-11-26), London, pages 563 - 567, XP055262130, ISSN: 0028-0836, DOI: 10.1038/nature14011
- LUCAS A. HORN ET AL: "CD3xPDL1 bi-specific T cell engager (BiTE) simultaneously activates T cells and NKT cells, kills PDL1<sup>+</sup> tumor cells, and extends the survival of tumor-bearing humanized mice", ONCOTARGET, vol. 8, no. 35, 29 August 2017 (2017-08-29), pages 57964 - 57980, XP055471264, DOI: 10.18632/oncotarget.19865
- B. BOYERINAS ET AL: "Antibody-Dependent Cellular Cytotoxicity Activity of a Novel Anti-PD-L1 Antibody Avelumab (MSB0010718C) on Human Tumor Cells", CANCER IMMUNOLOGY RESEARCH, vol. 3, no. 10, 26 May 2015 (2015-05-26), US, pages 1148 - 1157, XP055389536, ISSN: 2326-6066, DOI: 10.1158/2326-6066.CIR-15-0059
- FREEMAN ET AL.: "Engagement of the PD-1 Immunoinhibitory Receptor by a Novel B7 Family Member Leads to Negative Regulation of Lymphocyte Activation", JOURNAL OF EXPERIMENTAL MEDICINE, vol. 192, no. 7, 2 October 2000 (2000-10-02), pages 1027 - 1034, XP 002942545
- OSADA ET AL.: "CEA/ CD 3-Bispecific T cell -Engaging (BiTE) Antibody-Mediated T Lymphocyte Cytotoxicity Maximized by Inhibition of both PD1 and PD-L1`", CANCER IMMUNOLOGY, IMMUNOTHERAPY, vol. 64, 6 March 2015 (2015-03-06), pages 677 - 688, XP 055236106
- BUTTE ET AL.: "`PD-L1 Interacts Specifically with B7-1 to Inhibit T Cell Proliferation", IMMUNITY, vol. 27, 12 July 2007 (2007-07-12), pages 1 - 22, XP055555055
- BENNETT ET AL.: "Program Death-1 Engagement upon TCR Activation has Distinct Effects on Costimulation and Cytokine-Driven Proliferation: Attenuation of ICOS, IL -4, and IL -21, but not CD 28, IL -7, and IL -15 Responses", THE JOURNAL OF IMMUNOLOGY, vol. 170, 2003, pages 711 - 718, XP 055048008, DOI: doi:10.4049/jimmunol.170.2.711

## Description

### BACKGROUND OF THE INVENTION

### Technical Field

The present invention relates generally to the field of fusion proteins to be used to stimulate an immune response, and more specifically, a bispecific T-Cell engager recombinant polypeptide comprising a moiety that binds to CD3 on a T-cell and a moiety that binds to Programmed Death Ligand 1 (PDL1) on a tumor cell to counteract the immune tolerance of cancer cells.

### Related Art

The immune system provides the human body with a means to recognize and defend itself against microorganisms and substances recognized as foreign or potentially harmful. While passive immunotherapy of cancer with monoclonal antibodies and passive transfer of T-cells to attack tumor cells have demonstrated clinical efficacy, the goal of active therapeutic vaccination to induce these immune effectors and establish immunological memory against tumor cells has remained challenging. Several tumor-specific and tumor-associated antigens have been identified, yet these antigens are generally weakly immunogenic and tumors employ diverse mechanisms to create a tolerogenic environment that allows them to evade immunologic attack. Strategies to overcome such immune tolerance and activating robust levels of antibody and/or T-cell responses hold the key to effective cancer immunotherapy.

While existing bi-specific T-cell engagers have clinical efficacy, they do not reduce immune suppression and therefore are not therapeutic in all cancer patients. Thus, there is a need to develop a bi-specific T-cell engager that activates T-cells, induces cytotoxicity and kills tumor cells despite the presence of immune suppression mediated by PD1.

Roy S. Herbst et al., 2014, Nature, vol. 515(7528): 563-567 interrogates the PD-L1 expression on tumor cells. This document evaluates the safety, activity and biomarkers of PD-L1 inhibition using the engineered humanized antibody MPDL3280A.

The patent document WO 2015/112534 A2 discloses a bispecific antibody against CD3 and CEA, which activity can be further enhanced by addition of an antagonistic anti PD-L1 antibody.

The patent document WO 2016/034085 A1 discloses antibody drug conjugates binding PD-L1. Such immune-conjugates are effective in killing cancer cells in vivo.

The patent document WO 2016/020065A1 discloses a bispecific antibody for use in the treatment of a cancer disease, wherein said antibody binds CD3 and PD-L1.

The patent document WO 2013/079174 A1 discloses a method of treating cancer comprising administering to a subject in need thereof an effective amount of an anti-PD-L1 antibody which induces antibody dependent cell-mediated cytotoxicity (ADCC).

B. Boyerinas et al., 2015, Cancer Immunology Research, vol. 3(10):1148-1157 discloses a human IgG1 antibody which binds to and antagonized human PD-L1, known as avelumab.

### SUMMARY OF THE INVENTION

The present invention provides for a bispecific T-Cell engager (BiTE) recombinant protein useful in a variety of therapeutic methods for the treatment of cancer. Bi-specific T-cell engagers (BiTE) are genetically engineered recombinant proteins that simultaneously activate T-cells and induce the T cells to become cytotoxic through the T-cell receptor/CD3 complex and target the activated T-cells to tumor cells. The present invention provides methods of reducing growth and/or killing of cancer cells by counteracting immune tolerance of cancer cells, wherein T-cells remain active and inhibit the recruitment of regulatory cells that are known to suppress the immune system's response to the tumor. The present invention is also designed to concurrently activate naive T-cells through the binding of anti-CD3, while binding to target tumor cells or other cells expressing the inhibitory ligand PDL1.

In one aspect, the present invention provides for an anti-CD3/anti PDL1 bispecific fusion protein comprising a variable region of a heavy chain of an anti-CD3 antibody, a variable region of a light chain of an anti-CD3 antibody ad an amino acid linker positioned therebetween and attached to each anti-CD3 variable region and that binds to a CD3 receptor on a T-cell; and a variable region of a heavy chain of an anti- PDL1 antibody, a variable region of a light chain of an anti-PDL1 antibody and an amino acid linker positioned therebetween and attached to each anti-PDL1 variable region that binds to Programmed Death Ligand 1 (PDL1) on a tumor cell to activate T-cells through CD3 and simultaneously redirecting T-cells to PDL1+ tumor cells, neutralizing PD1-mediated suppression, and killing the tumor cells, wherein the variable region of the heavy chain of the anti-PDL1 antibody is linked through an amino acid linker to the variable region of the heavy chain of the anti-CD3 antibody wherein the amino acid linker is sufficient number of amino acid residues for binding of the anti-CD3/anti-PDL1 bispecific fusion protein to individual targets, wherein the variable region of the light chain of the PDL1 antibody and the variable region of the heavy chain of the anti-PDL1 antibody consists of amino acid residues SEQ ID NOs: 5 and 6 respectively and wherein the variable region of the light chain of the anti-CD3 antibody and the variable region of the heavy chain of the anti-CD3 antibody consists of amino acid residues SEQ ID NOs: 7 and 8 respectively.

In the alternative, not covered by the present invention, the anti-CD3 moiety and the anti-PDL1 moiety that counteract immune tolerance of cancer cell may be bound directly to each other without a linker. The anti-CD3/anti-PDL1 bispecific fusion protein of the present invention is useful for binding to a cancer cell receptor (PDL1) and reducing the ability of cancer cells to avoid an immune response, while concurrently activating and directing T-cells (CD3) to the targeted tumor cell and inducing death of such cancer cell.

The present invention is based on preparing bispecific proteins by expression of polynucleotides encoding the bispecific proteins that counteract or reverse immune tolerance of cancer cells and simultaneously activate T-cells. Cancer cells are able to escape elimination by chemotherapeutic agents or tumor-targeted antibodies via specific immunosuppressive mechanisms in the tumor microenvironment and such ability of cancer cells is recognized as immune suppression. By counteracting tumor-induced immune suppression, the present invention provides effective compositions comprising the anti-CD3/anti-PDL1 bispecific fusion protein of the invention for cancer treatment, optionally in combination with another existing cancer treatment. The present invention provides strategies to counteract tumor-induced immune suppression by activating and leveraging T-cell-mediated adaptive antitumor immunity against resistant cancer cells.

The present disclosure provides for a method of treating cancer and/or killing cancerous tumor cells, the method comprising:
administering to a subject an anti-CD3/anti-PDL1 bispecific fusion protein comprising an anti-CD3 antibody ,fragments thereof, or single chain variable fragments that binds to a CD3 receptor on a T-cell and an anti PDL1 antibody, fragments thereof, or single chain variable fragments that binds to PDL1 on a tumor cell, wherein the binding to CD3 on CD4⁺ or CD8⁺ T-cell activates the T-cells that are cytotoxic for PDL1⁺ tumor cells, thereby inducing death of the PDL1⁺ tumor cells.

Notably the activated T-cells cells have been found to be cytotoxic for PDL1⁺ human melanoma, chronic myelogenous leukemia (CML), breast cancer, and non-small cell lung cancer cells (NSCLC).

The amino acid residues for the light and heavy chains of the anti-PDL1 antibody are SEQ ID NOs. 5 and 6, respectively and the light and heavy chains of the anti-CD3 antibody are SEQ ID NOs. 7 and 8, respectively. The linker may include amino acid residues SEQ ID NOs. 12, 14 or 16. The heavy or light chain of the anti-PDL1 antibody are linked to the light or heavy chain of an anti-CD3 antibody. The heavy chain of the anti-PDL1 antibody is linked to heavy chain of an anti-CD3 antibody.

The present disclosure provides for genes encoding for a bispecific comprising an anti-CD3 antibody or fragments thereof and an anti-PDL1 antibody or fragments thereof for treating cancer in a human subject for expression in a human subject. Preferably the genes comprise sequences selected from SEQ ID NOs: 1 to 4.

The present disclosure provides for a method of treating cancer with an anti-CD3/anti-PDL1 bispecific fusion protein in a subject, the method comprising:
a. providing at least one recombinant vector comprising nucleotide sequences that encode for an anti-CD3 antibody or fragments thereof and an anti-PDL1 antibody or fragments thereof wherein the nucleotide sequences comprise SEQ ID NOs: 1 to 4; and
b. administering the recombinant vector to the subject under conditions such that said nucleotide sequences are expressed at a level which produces a therapeutically effective amount of the encoded bispecific fusion protein in the subject, wherein the expressed amino acid residues for the bispecific fusion protein comprises SEQ ID NOs. 7 and 8 for the light and heavy chains of the anti-CD3 antibody respectively and SEQ ID NOs. 5 and 6 for the light and heavy chains of the anti-PDL1 antibody respectively.

The present disclosure provides a recombinant host cell transfected with a polynucleotide that encodes an anti-CD3/anti-PDL1 bispecific fusion protein of the present invention.

In a still further aspect, the present invention contemplates a process of preparing a bispecific fusion protein of the present invention comprising:
a. transfecting a host cell with polynucleotide sequences that encode the variable regions of the heavy and light chains of the anti-CD3/anti-PDL1 bispecific fusion protein to produce a transformed host cell, wherein the polynucleotide sequences encode the variable regions of the light and heavy chains of the anti-CD3 antibody and the variable region of the light and heavy chains of the anti-PDL1 antibody and linkers positioned between the variable regions and having a nucleotide sequence of SEQ ID NO: 9; and
b. maintaining the transformed host cell under biological conditions sufficient for expression of the bispecific fusion protein having and amino acid sequence of SEQ ID NO: 10.

The present disclosure provides a method of preventing or treating a neoplastic disease. The method includes administration to a subject in need thereof a bispecific fusion protein of the invention in combination with another anticancer therapy, in one aspect, the anticancer therapy includes a chemotherapeutic molecule, antibody, small molecule kinase inhibitor, such as tyrosine kinase inhibitors (TKIs), hormonal agent or cytotoxic agent. The anticancer therapy may also include ionizing radiation, ultraviolet radiation, cryoablation, thermal ablation, or radiofrequency ablation.

The present disclosure provides for a method of preparing a therapeutically active anti-CD3/anti-PDL1 bispecific fusion protein comprising an anti-CD3 moiety and an anti-PDL1 moiety, the method comprising;
a. preparing a nucleotide sequence of the anti-CD3/anti-PDL1 bispecific fusion protein;
b. cloning the nucleotide sequence of the anti-CD3/anti-PDL1 bispecific fusion protein in a host cell capable of transient or continued expression;
c. growing the host cell in a media under suitable conditions for growing and allowing the host cell to express the cloned anti-CD3/anti-PDL1 bispecific fusion protein; and
d. subjecting the expressed bispecific fusion protein to purification and optionally checking the bi-specific binding capabilities of the anti-CD3/anti-PDL1 bispecific fusion protein to its targets.

Such tests may include in-vitro test such as ELISA or NK/T-cell binding assays to validate bi-functional target binding or immune cell stimulation.

Notably once the anti-CD3/antiPDL1 bispecific fusion protein demonstrates the desired bi-specificity, such anti-CD3/antiPDL1 bispecific fusion protein is selected for subcloning into a stable cell line for larger scale expression and purification. Such stable cell lines are previously disclosed, such as a mammalian cell line, including but not limited to HEK293, CHO or NSO or a bacterial cell line, including but not limited to E.coli, or in yeast, including but not limited to saccharomyces cerevisiae.

The present disclosure provides for use of an anti-CD3/anti-PDL1 bispecific fusion protein comprising an anti-CD3 moiety that binds to CD3 on a T-cell and an anti-PDL1 moiety that binds to Programmed Death Ligand 1 (PDL1) on a tumor cell in a medicament for the treatment of cancer.

The present disclosure provides for a method of killing cancerous tumor cells, the method comprising:
a. contacting a cancerous tumor cell with an anti-CD3/antiPDL1 bispecific fusion protein comprising an anti-CD3 antibody or fragments thereof that binds to a CD3 receptor on a T-cell and an anti- PDL1 antibody or fragments thereof that binds to Programmed Death Ligand 1 (PDL1⁺) on a tumor cell, wherein the binding to the CD3 receptor on the T-cell activates the T-cells that are cytotoxic for a PDL1⁺ tumor cell, such as activating CD4⁺ and CD8⁺ T-cells and thereby inducing increased death of the PDL1⁺ tumor cell. Any reference to a method of treatment practised on the human or animal body is to be interpreted as substances or compositions for use in such treatment.

Other features and advantages of the invention will be apparent from the following detailed description, drawings and claims.

### BRIEF DESCRIPTION OF THE FIGURES

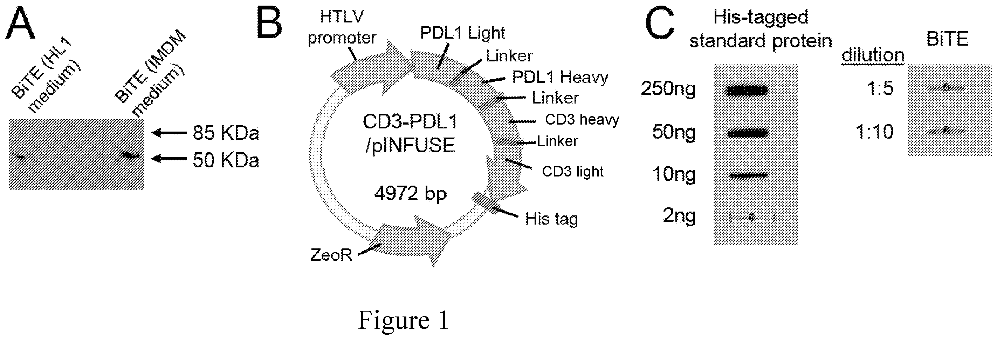

Figures 1A, 1B and 1C show CHO cells transfected with the anti-CD3/anti-PDL1 fusion protein produce ~1.2 µg protein/10⁷ cells/48 hrs. Figure 1A shows that the anti-CD3/anti-PDL1 fusion protein (referred to as BiTE in all figures) of the present invention is a ~55kD secreted protein. CHO cells were transfected with nucleotide sequences encoding the anti-CD3/anti-PDL1 fusion protein and were incubated at 37°C 5% CO₂ in serum-free HL1 or serum-containing IMDM medium. Supernatants were collected, concentrated using 10kD spin columns, electrophoresced by SDS-PAGE, and western blotted with an anti-His antibody. Figure 1B shows the construct used to transfect CHO cells. Figure 1C shows 1 x 10⁷ transfectants were incubated in 100 ml of serum-free HL-1 medium for 48 hrs. Supernatants were collected and remaining cells were removed by centrifugation. Supernatants were concentrated to 1.5 ml using 10kD spin columns. Concentrated supernatant was diluted 1:5 and 1:10 with PBS and slot blotted along with dilutions of a control his-tagged protein. Dried blots were probed with an anti-his antibody. The anti-CD3/anti-PDL1 fusion protein was quantified using Image J software.

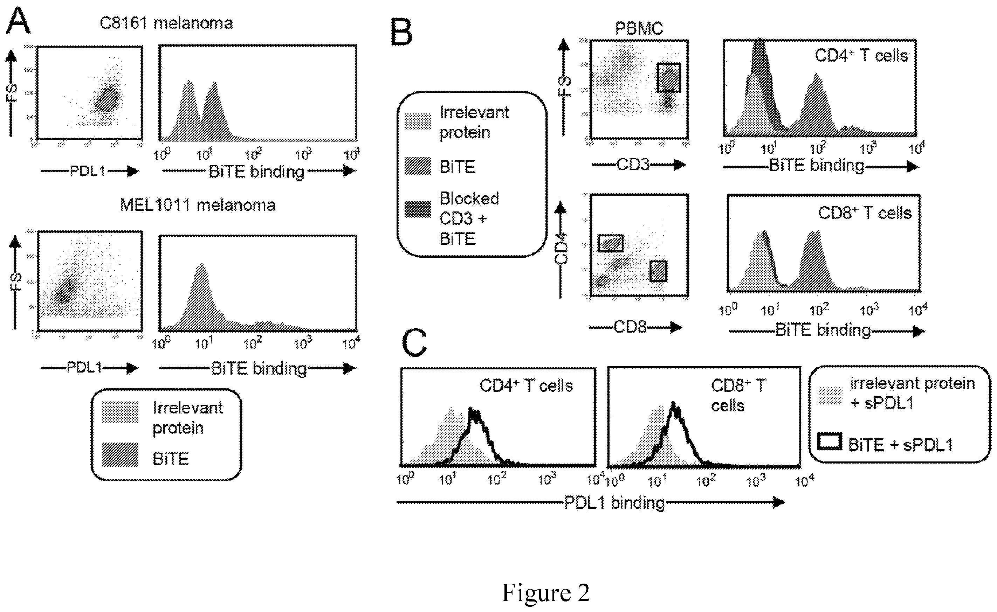

Figures 2A, B and C show that the anti-CD3/anti-PDL1 fusion protein (referred to as BiTE in all figures) of the present invention simultaneously and specifically binds to PDL1⁺ human tumor cells and CD3⁺ human T-cells. Figure 2A shows the anti-CD3/anti-PDL1 fusion protein of the present invention binds specifically to PDL1⁺ human tumor cells. C8161 and MEL1011 melanoma cells were stained with antibodies to PDL1 (left panels). PDL1⁺ C8161 and PDL1⁻ MEL1011 cells were incubated with the anti-CD3/anti-PDL1 fusion protein of the present invention or an irrelevant control protein and stained with anti-His antibody to detect binding of the anti-CD3/anti-PDL1 fusion protein of the present invention (right panels). Figure 2B shows that the anti-CD3/anti-PDL1 fusion protein of the present invention binds specifically to CD3⁺ human T-cells. PBMC from healthy human donors were stained with antibodies to CD4 and CD8 followed by addition of the anti-CD3/anti-PDL1 fusion protein or irrelevant protein, and then antibody to His to detect the binding of the anti-CD3/anti-PDL1 fusion protein. Some PBMC were first blocked with antibodies to CD3 to prevent binding of the anti-CD3/anti-PDL1 fusion protein. Figure 2C shows that the anti-CD3/anti-PDL1 fusion protein simultaneously binds CD3 and PDL1. PBMC were stained for CD4 and CD8 as in panel B, and then stained with either the anti-CD3/anti-PDL1 fusion protein of the present invention or an irrelevant protein followed by soluble PDL1 (sPDL1). PDL1 binding was detected with antibody to PDL1. Data are representative of >3 independent experiments with multiple batches of the anti-CD3/anti-PDL1 fusion protein of the present invention.

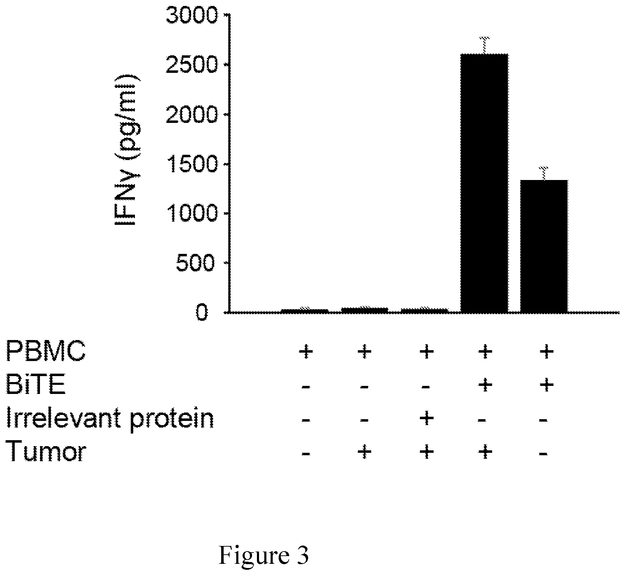

Figure 3 shows that the anti-CD3/anti-PDL1 fusion protein (BiTE) induces PBMC from healthy human donors to produce IFNγ in the presence of human melanoma (C8161) cells. Healthy donor PBMC (1.2 x 10⁶ cells) were incubated with the anti-CD3/anti-PDL1 fusion protein (6.5 ng/ml), C8161 human melanoma cells, and/or an irrelevant recombinant protein (Troy-Fc). Following a 48 hr incubation, supernatants were analyzed for IFNγ. PBMC to tumor cell ratio was 20:1. Data are representative of 1 of 2 independent experiments.

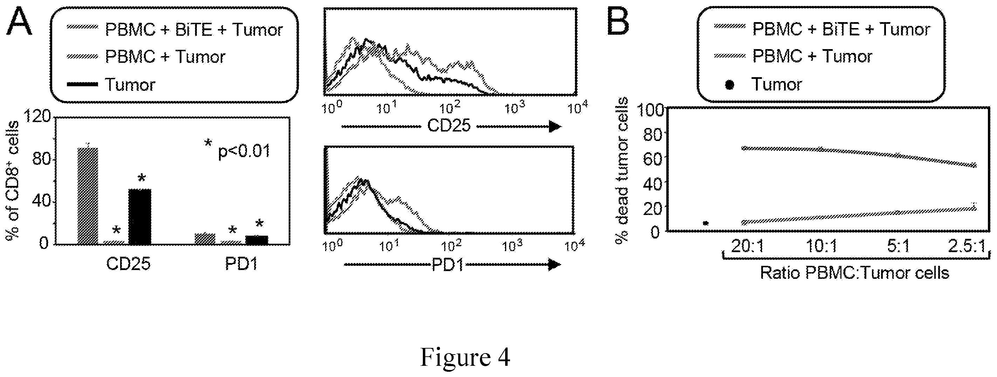

Figures 4 A and B show that the anti-CD3/anti-PDL1 fusion protein (BiTE) of the present invention activates human T-cells that are cytotoxic for PDL1⁺ human melanoma cells. PBMC from a healthy human donor were incubated at varying effector to target ratios with Cell Tracker violet-labeled PDL1⁺ C8161 human melanoma cells with or without (5.2 ng/ml). After a 48 hr incubation the cells were harvested. Figure 4A shows flow profiles for CD25 and PD1 staining for representative samples; bar graph shows the average of three replicates per sample. One aliquot of cells was labeled with antibodies to CD8, CD25, and PD1. CD8⁺ T-cells were gated and analyzed for the activation markers CD25 and PD1. In Figure 4 B, a separate aliquot of cells was stained with 7AAD to identify dead cells. % dead tumor cells = [dead tumor cells (violet⁺7AAD⁺)/total tumor cells (violet⁺)] x 100%. Values are the average of triplicates per sample. Error bars indicate standard deviations. Note: The actual ratio of T-cells to tumor cells is less than the indicated ratio since PBMC contain other cells in addition to T-cells.

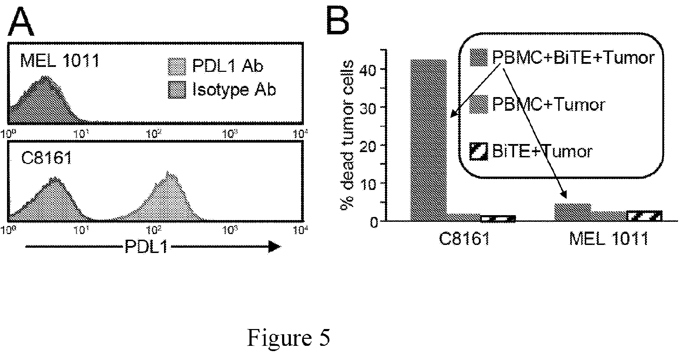

Figures 5 A and B show that the anti-CD3/anti-PDL1 fusion protein (BiTE) of the present invention is cytotoxic for human PDL1⁺ human tumor cells and does not kill PDL1⁻ tumor cells. Figure 5A shows the results of C8161 and MEL1011 human melanoma cells that were stained with antibodies to PDL1 and analyzed by flow cytometry. Figure 5B shows the results when PBMC from healthy donors were incubated ± violet-labeled human PDL1⁺ or PDL1⁻ tumor cells ± anti-CD3/anti-PDL1 fusion protein as in Figure 4 and analyzed for % dead tumor cells. PBMC:tumor cell ratio is 20:1. CD8:tumor cell ratio is ~4:1. Data are from 1 of 3 independent experiments.

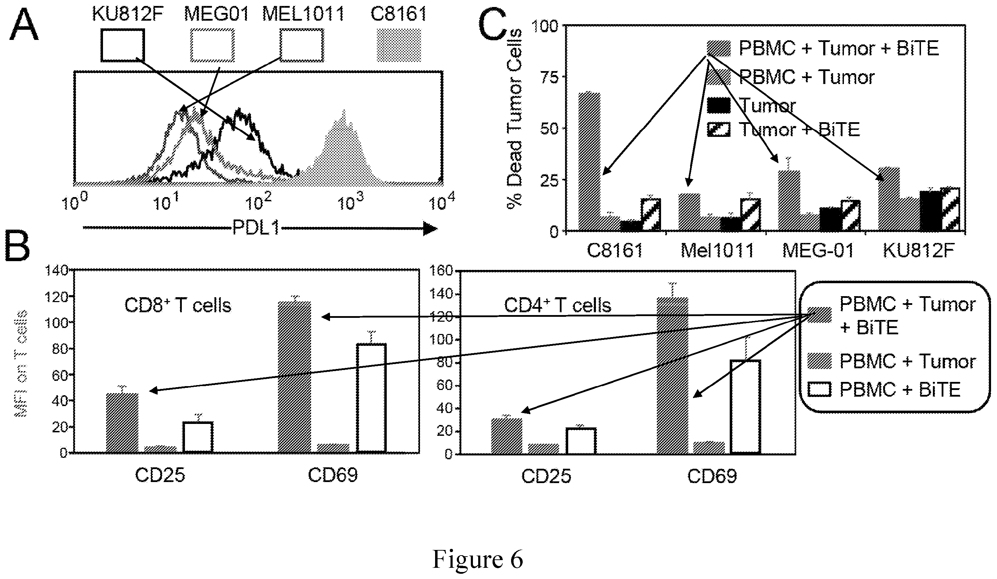

Figures 6 A, B and C show that anti-CD3/anti-PDL1 fusion protein (BiTE) of the present invention activates T-cells and is cytotoxic for chronic myelogenous leukemia (CML) cells. Figure 6A shows that approximately 20-25% of CML MEG-01 and KU812F cells are PDL1⁺ following treatment with IFNγ. Tumor cells were either untreated (melanomas C8161 and MEL1011) or incubated for 48 hrs with 200 units IFNγ (human CML KU812F and MEG01) and stained with antibody to PDL1. Figure 6B shows that the anti-CD3/anti-PDL1 fusion protein of the present invention activates CD4⁺ and CD8⁺ T-cells in the presence of PDL1⁺ tumor cells. PBMC incubated with Cell Tracker violet-labeled C8161 cells plus the anti-CD3/anti-PDL1 fusion protein were stained with antibodies to CD3, CD4, CD8, and for activation markers CD25 and CD69. MFI = mean fluorescence intensity. Figure 6C shows that the anti-CD3/anti-PDL1 fusion protein of the present invention kills ~25% of the CML cells. Violet-labeled C8161 tumor cells of panel B were analyzed for % dead tumor cells.

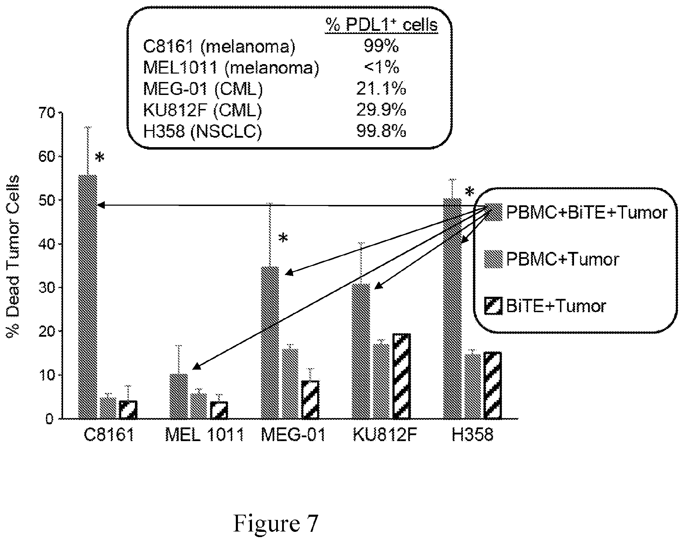

Figure 7 shows that the anti-CD3/anti-PDL1 fusion protein (BiTE) of the present invention activates T-cells that are cytotoxic for PDL1⁺ human melanoma, chronic myelogenous leukemia CML and non-small cell lung cancer cells. PBMC from healthy donors were incubated ± violet-labeled human tumor cells ± the anti-CD3/anti-PDL1 fusion protein of the present invention as in Figure 4 and analyzed for % dead tumor cells. PBMC:tumor cell ratio is 20:1. CD8:tumor cell ratio is ~4:1. Data are the average + SD of 4, 4, 3, and 2 independent experiments for C8161, MEL1011, MEG-01, KU812F, and H358 cells, respectively. * indicates the PBMC+ anti-CD3/anti-PDL1+ tumor value is statistically significantly different from the PBMC+tumor and the anti-CD3/anti-PDL1 + tumor values at p<0.05.

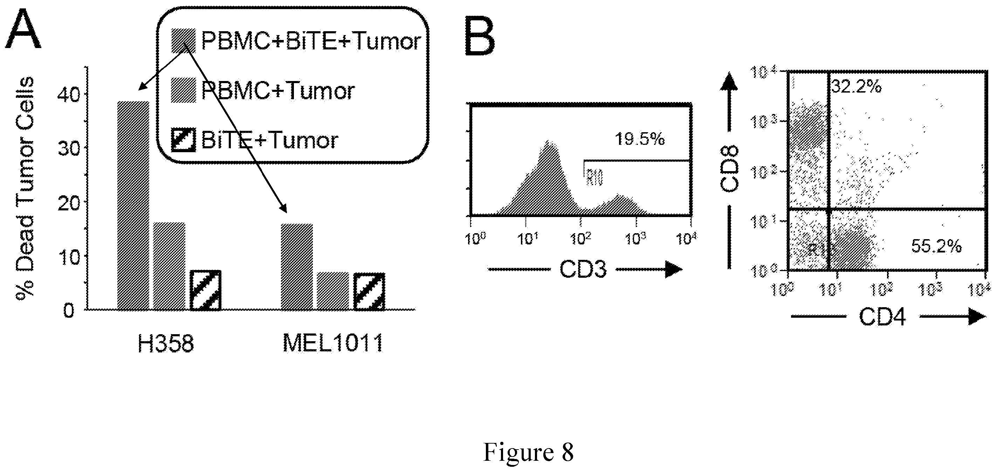

Figures 8 A and B shows that the anti-CD3/anti-PDL1 fusion protein (BiTE) activates PBMC from a small cell lung cancer patient that are cytotoxic for PDL1⁺ lung cancer cells. PBMC from a SCLC patient were incubated ± violet-labeled human PDL1⁺ (H358) or PDL1⁻ (MEL1011) tumor cells ± the anti-CD3/anti-PDL1 fusion protein at a 20:1 ratio of PBMC:tumor cells as in Figure 4. In Figure 8A cells were stained with the viability dye 7AAD and the Cell tracker violet stained tumor cells were gated and analyzed for % dead cells. The ratio of CD8 + T-cells to tumor cells is 1.29:1; the ratio of CD4⁺ T-cells to tumor cells is 2.17:1. Figure 8B shows the percent of CD3⁺ cells that are CD4⁺ or CD8⁺. Separate aliquots of PBMC were labeled with antibodies to CD3, CD4 and CD8, and the CD3 gated cells were analyzed for percent CD3⁺CD4⁺ and CD3⁺CD8⁺ cells.

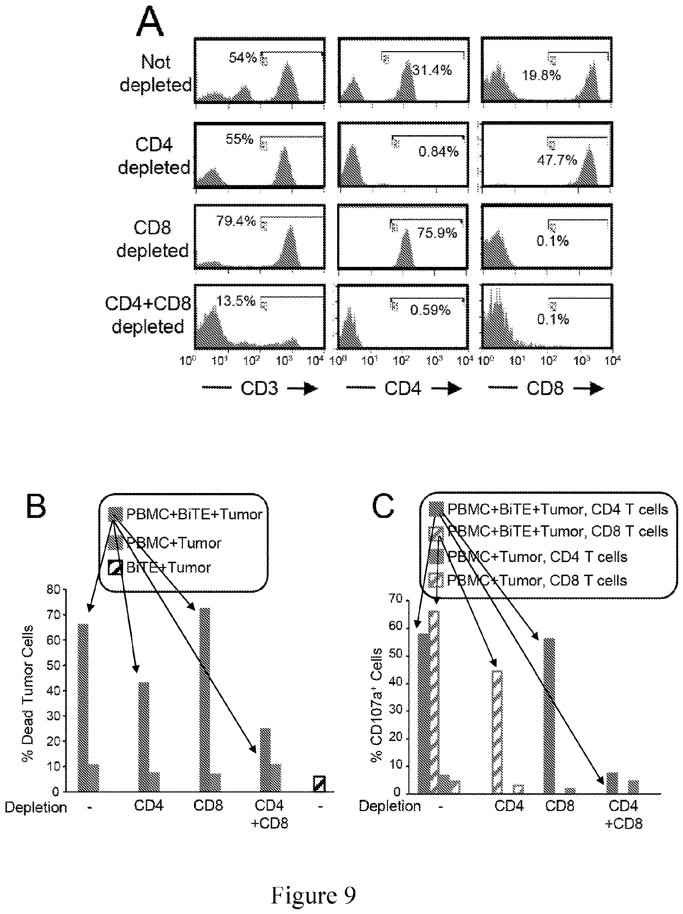

Figures 9 A, B and C show that the anti-CD3/anti-PDL1 fusion protein (BiTE) activates both CD4⁺ and CD8⁺ cytotoxic T-cells. In Figure 9A, PBMC from a healthy human donor were either undepleted, or depleted for CD4⁺ T-cells, CD8⁺ T-cells, or CD4⁺ + CD8⁺ T-cells. Depleted populations were stained with antibodies to CD3, CD4, and CD8, and the CD3⁺ cells gated and analyzed for CD4 and CD8 expression. Values are the percent of cells in the total PBMC population. In Figure 9B, violet-labeled human PDL1⁺ (H358) or PDL1⁻ (MEL1011) tumor cells were incubated with the depleted or not depleted PBMC ± the anti-CD3/anti-PDL1 and analyzed for percent dead tumor cells as in Figure 4. PBMC:tumor cell ratio is 20:1. CD8:tumor cell ratio is 5.2:1; CD4:tumor cell ratio is 7.8:1. Data are the average of two experiments. In Figure 9C, the cells of Figure 9A were stained with antibodies to CD4, CD8, and CD107a (granule identifying cytotoxic T-cells) and the CD4 and CD3 cells gated and analyzed for CD107a expression. Data are representative of 1 of 2 independent experiments.

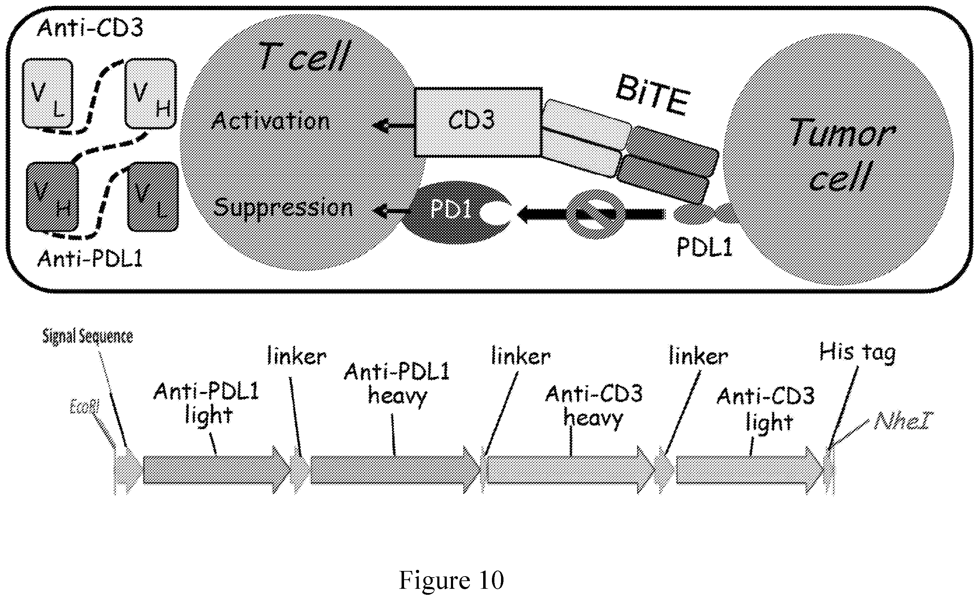

Figure 10 A shows the anti-CD3/anti-PDL1 bispecific fusion protein (BiTE) that binds to both CD3 on a T-cell and binds to PDL1 on a tumor cell. Figure 10 B shows the gene map for a recombinant construct consisting of VL+VH segments of anti-PDL1 and anti-CD3 antibodies.

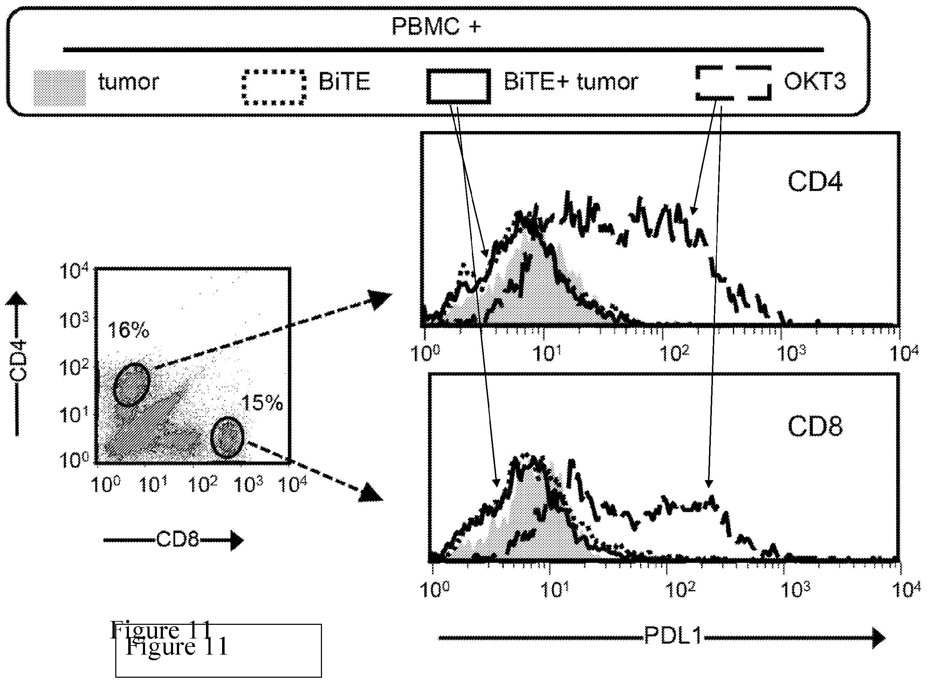

Figure 11 shows that the anti-CD3/anti -PDL1 bispecific fusion protein (BiTE) of the present invention does not induce PDL1 expression on activated T-cells, whereas the OKT3 antibody does induce PDL1 on activated T-cells.

### DETAILED DESCRIPTION OF THE INVENTION

Recent clinical trials have demonstrated that a patient's immune system has the capacity to cure cancer patients through T-cell-mediated killing of tumor cells.¹⁻³ However, only ~19-30% of patients with limited types of advanced tumors respond to existing immunotherapeutics. Since many solid and hematologic tumors are immune suppressive due to the expression of the co-inhibitory molecule Programmed Death Ligand 1 (PDL1)⁴, optimal immunotherapies must simultaneously accomplish three functions: (i) activate cytotoxic CD8⁺ T-cells (CTL); (ii) neutralize PD1 suppression; and (iii) target activated CTL to kill the tumor cells. Existing immunotherapies mediate one or two of these functions, but do not mediate all three simultaneously. The presently claimed bispecific T-cell Engager fusion protein (anti-CD3/anti-PDL1 (BiTE)) has the potential to simultaneously mediate all three functions. Because most tumors express PDL1, the presently claimed bispecific fusion protein has the potential to treat any type of cancer.

The practice of the present invention will employ, unless otherwise indicated, conventional techniques of immunology, molecular biology, microbiology, cell biology and recombinant DNA, which are within the skill of the art. See, e.g., Sambrook, et al. MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)).

### Definitions

Unless otherwise defined, all technical and scientific terms used herein have the meaning commonly understood by one of ordinary skill in the art to which this invention belongs. The terminology used herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. The following terms have the meanings given:

The term "polynucleotide" as used herein means a sequence of nucleotides connected by phosphodiester linkages. Polynucleotides are presented herein in the direction from the 5' to the 3' direction. A polynucleotide of the present invention can be a deoxyribonucleic acid (DNA) molecule or ribonucleic acid (RNA) molecule. Where a polynucleotide is a DNA molecule, that molecule can be a gene or a cDNA molecule. Nucleotide bases are indicated herein by a single letter code: adenine (A), guanine (G), thymine (T), cytosine (C), inosine (I) and uracil (U). A polynucleotide of the present invention can be prepared using standard techniques well known to one of skill in the art.

The term "transfection" of a cell as used herein means that genetic material is introduced into a cell for the purpose of genetically modifying the cell. Transfection can be accomplished by a variety of means known in the art, such as transduction or electroporation.

The term "transgene" is used in a broad sense to mean any heterologous nucleotide sequence incorporated in a vector for expression in a target cell and associated expression control sequences, such as promoters. It is appreciated by those of skill in the art that expression control sequences will be selected based on ability to promote expression of the transgene in the target cell. An example of a transgene is a nucleic acid encoding a bispecific fusion protein of the present invention.

The term "expression vector" as used herein means a vector containing a nucleic acid sequence coding for at least part of a gene product capable of being transcribed. Expression vectors can contain a variety of control sequences, which refer to nucleic acid sequences necessary for the transcription and possibly translation of an operatively linked coding sequence in a particular host organism. In addition to control sequences that govern transcription and translation, vectors and expression vectors may contain nucleic acid sequences that serve other functions as well. The term also includes a recombinant plasmid or virus that comprises a polynucleotide to be delivered into a host cell, either in vitro or in vivo. Preferably the host cell is a transient cell line or a stable cell line and more preferably a mammalian host cell and selected from, but not limited to, the group consisting of HEK293, CHO and NSO or a bacterial cell line, including but not limited to E.coli, or in yeast, including but not limited to saccharomyces cerevisiae.

The term "subject," as used herein means a human or vertebrate animal including a dog, cat, horse, cow, pig, sheep, goat, chicken, monkey, rat, and mouse.

The term "therapeutically effective amount" as used herein means the amount of the subject compound that will elicit the biological or medical response of a tissue, system, animal or human that is being sought by the researcher, veterinarian, medical doctor or other clinician.

The term "pharmaceutically acceptable" as used herein means the carrier, diluent or excipient must be compatible with the other ingredients of the formulation and not deleterious to the recipient thereof.

The terms "cancer" or "cancerous" as used herein refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small-cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatome, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, prostate cancer, skin cancer, ocular cancer, brain cancer, lymphoma, leukemia, melanoma as well as head and neck cancer.

The term "recombinant" as used herein means a genetic entity distinct from that generally found in nature. As applied to a polynucleotide or gene, this means that the polynucleotide is the product of various combinations of cloning, restriction and/or ligation steps, and other procedures that result in the production of a construct that is distinct from a polynucleotide found in nature.

The term "peptide," "polypeptide" and "protein" are used interchangeably to denote a sequence polymer of at least two amino acids covalently linked by an amide bond.

The term "administering" as used herein is defined as the actual physical introduction of the composition into or onto (as appropriate) the host subject. Any and all methods of introducing the composition into the subject are contemplated according to the present disclosure; the method is not dependent on any particular means of introduction and is not to be so construed. Means of introduction are well-known to those skilled in the art, and preferably, the composition is administered subcutaneously or intratumorally. One skilled in the art will recognize that, although more than one route can be used for administration, a particular route can provide a more immediate and more effective reaction than another route. Local or systemic delivery can be accomplished by administration comprising application or instillation of the immunovaccines into body cavities, inhalation or insufflation of an aerosol, or by parenteral introduction, comprising intramuscular, intravenous, intraportal, intrahepatic, peritoneal, subcutaneous, intracranial, including intracerebral, intrathecal, intratumoral, or intradermal administration.

Although chemotherapeutic agents can induce "immunogenic" tumor cell death and facilitate cross-presentation of antigens by dendritic cells, tumors create a tolerogenic environment that allows them to suppress the activation of innate and adaptive immune responses and evade immunologic attack by immune effector cells. The present invention provides strategies to counteract tumor-induced immune tolerance in the tumor microenvironment and can enhance the antitumor efficacy of chemotherapy by activating and leveraging T-cell-mediated adaptive antitumor immunity against disseminated cancer cells.

The present invention is based on the discovery that the bispecific fusion proteins of the present invention can counteract or reverse immune tolerance of cancer cells. Cancer cells are able to escape elimination by chemotherapeutic agents or tumor-targeted antibodies via specific immunosuppressive mechanisms in the tumor microenvironment and such ability of cancer cells is recognized as immune tolerance. By counteracting tumor-induced immune tolerance, the present invention provides effective compositions and for cancer treatment, optional in combination with another existing cancer treatment.

The present invention provides compositions and methods for producing bispecific fusion proteins that counteract immune tolerance in the tumor microenvironment and promote T-cell-mediated adaptive antitumor immunity for maintenance of durable long-term protection against recurrent or disseminated cancers. These bispecific fusion proteins are designed to facilitate effective long term T-cell-mediated immune responses against tumor cells by increasing activation and proliferation of antitumor CD8+ T-cells and CD4+ T-cells by negating immune suppression mediated by regulatory T-cells and myeloid suppressor cells. These antitumor immune responses may be activated in tandem with the sensitization of tumor cells to immune effector-mediated cytotoxicity, thereby establishing a positive feedback loop that augments tumor cytoreduction and reinforces adaptive antitumor immunity.

The bispecific fusion proteins of the invention provide the ability to disrupt immunosuppressive networks in the tumor microenvironment. Tumors employ a wide array of regulatory mechanisms to avoid or suppress the immune response. Cancer cells actively promote immune tolerance in the tumor microenvironment via the expression of cytokines and molecules that inhibit the differentiation and maturation of antigen-presenting dendritic cells (DC). The immunosuppressive cytokines and ligands produced by tumor cells include PDL1 and such increase is correlated with a reduction of survival of patients with diverse types of cancers. The bispecific fusion proteins of the present invention inhibit key immunosuppressive molecules expressed by the targeted tumor.

The present disclosure provides a method of preventing or treating a neoplastic disease. The method includes administration to a subject in need thereof the bispecific fusion protein of the present invention in combination with another anticancer therapy, wherein the anticancer therapy is a chemotherapeutic molecule, antibody, small molecule kinase inhibitor, hormonal agent, cytotoxic agent, targeted therapeutic agent, anti-angiogenic agent, ionizing radiation, ultraviolet radiation, cryoablation, thermal ablation, adoptive cell therapy, hematopoietic stem cell transplantation or bone marrow transplantation, or radiofrequency ablation.

As used herein, the term "antibody" includes natural or artificial mono- or polyvalent antibodies including, but not limited to, polyclonal, monoclonal, multispecific, human, humanized or fusion antibodies, single chain antibodies, Fab fragments. F(ab') fragments, fragments produced by a Fab expression library, anti-idiotypic (anti-Id) antibodies (including, e.g., anti-Id antibodies to antibodies of the invention), and epitope-binding fragments of any of the above. The antibody may be from any animal origin including birds and mammals. **In** one aspect, the antibody is, or derived from, a human, murine (e.g., mouse and rat), donkey, sheep, rabbit, goat, guinea pig, camelid, horse, or chicken. Further, such antibody may be a humanized version of an antibody. The antibody herein specifically include a "fusion" antibody in which a portion of the heavy and/or light chain is identical with or homologous to corresponding sequences in antibodies derived from one type of antibody, while the remainder of the chain(s) is identical with or homologous to corresponding sequences in antibodies belonging to another antibody type, as well as fragments of such antibodies, so long as they exhibit the desired biological activity.

Various methods have been employed to produce antibodies. Hybridoma technology, which refers to a cloned cell line that produces a single type of antibody, uses the cells of various species, including mice (murine), hamsters, rats, and humans. Another method to prepare an antibody uses genetic engineering including recombinant DNA techniques. For example, antibodies made from these techniques include, among others, fusion antibodies and humanized antibodies. A fusion antibody combines DNA encoding regions from more than one type of species. For example, a fusion antibody may derive the variable region from a mouse and the constant region from a human. A humanized antibody comes predominantly from a human, even though it contains nonhuman portions. Like a fusion antibody, a humanized antibody may contain a completely human constant region. But unlike a fusion antibody, the variable region may be partially derived from a human. The nonhuman, synthetic portions of a humanized antibody often come from Complementarity Determining Regions in murine antibodies. In any event, these regions are crucial to allow the antibody to recognize and bind to a specific antigen.

An antibody fragment can include a portion of an intact, antibody, e.g. including the antigen-binding or variable region thereof. Examples of antibody fragments include Fab, Fab', F(ab')2, and Fv fragments; Fc fragments or Fc-fusion products; diabodies; linear antibodies; single-chain antibody molecules; and multispecific antibodies formed from antibody fragment(s). An intact antibody is one which includes an antigen-binding variable region as well as a light chain constant domain (CL) and heavy chain constant domains, CH1, CH2 and CH3. The constant domains may be native sequence constant domains (e.g., human native sequence constant domains) or amino acid sequence variant thereof or any other modified Fc (e.g. glycosylation or other engineered Fc).

In the present invention, a linker is used for linking the heavy and light chains of the respective antibodies. In one embodiment, a linker is (GGGGS)n wherein n is 1, 2, 3, 4, 5, 6, 7, or 8. For example, GGGGSGGGGSGGGGS (SEQ ID NO: 12). In another embodiment, a linker is (GGS)n wherein n is 1, 2, 3, 4, 5, 6, 7, or 8. Another linker may include, for example, GGSGGSGGSGGSGGVD (SEQ ID NO: 16). In various aspects, the length of the linker may be modified to optimize binding of the two antibodies to their respective binding sites.

The bispecific fusion proteins of the present invention may be synthesized by conventional techniques known in the art, for example, by chemical synthesis such as solid phase peptide synthesis. Such methods are known to those skilled in the art. In general, these methods employ either solid or solution phase synthesis methods, both well known in the art. Specifically, the methods comprise the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complementary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups and any solid support are removed either sequentially or concurrently to afford the final polypeptide. By simple modification of this general procedure, it is possible to add more than one amino acid at a time to a growing chain, for example, by coupling (under condition that do not racemize chiral centers) a protected tripeptide with a properly protected dipeptide to form, after deprotection, a pentapeptide.

Typical protecting groups include t-butyloxycarbonyl (Boc), 9-fluorenylmethoxycarbonyl (Fmoc), benxyloxycarbonyl (Cbz), p-toluenesulfonyl (Tos); 2,4-dinitrophenyl, benzyl (Bzl), biphenylisopropyloxy-carboxycarbonyl, cyclohexyl, isopropyl, acetyl, o-nitrophenylsulfonyl, and the like. Of these, Boc and Fmoc are preferred.

Typical solid supports are generally cross-linked polymeric materials. These include divinylbenzene cross-linked styrene-based polymers, for example, divinylbenzene-hydroxymethylstyrene copolymers, divinylbenzene-chloromethylstyrene copolymers, and divinylbenzene-benzhydrylaminopolystyrene copolymers. The divinylbenzene-benzhydrylaminopolystyrene copolymers, as illustrated herein using p-methyl-benzhydrylamine resin, offers the advantage of directly introducing a terminal amide functional group into the peptide chain, which function is retained by the chain when the chain is cleaved from the support.

In one method, the polypeptides are prepared by conventional solid phase chemical synthesis on, for example, an Applied Biosystems, Inc. (ABI) 430A peptide synthesizer using a resin that permits the synthesis of the amide peptide form and using t-Boc amino acid derivatives (Peninsula Laboratories, Inc.) with standard solvents and reagents. Polypeptides containing either L- or D-amino acids may be synthesized in this manner. Polypeptide composition is confirmed by quantitative amino acid analysis and the specific sequence of each peptide may be determined by sequence analysis.

Preferably, the polypeptides can be produced by recombinant DNA techniques by synthesizing DNA encoding the desired polypeptide. Once coding sequences for the desired polypeptides have been synthesized or isolated, they can be cloned into any suitable vector for expression. Numerous cloning vectors are known to those of skill in the art, and the selection of an appropriate cloning vector is a matter of choice. The gene can be placed under the control of a promoter, ribosome binding site (for bacterial expression) and, optionally, an operator (collectively referred to herein as "control" elements), so that the DNA sequence encoding the desired polypeptide is transcribed into RNA in the host cell transformed by a vector containing this expression construction. The coding sequence may or may not contain a signal peptide or leader sequence. Heterologous leader sequences can be added to the coding sequence that causes the secretion of the expressed polypeptide from the host organism. Other regulatory sequences may also be desirable which allow for regulation of expression of the protein sequences relative to the growth of the host cell. Such regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

The control sequences and other regulatory sequences may be ligated to the coding sequence prior to insertion into a vector, such as the cloning vectors described above. Alternatively, the coding sequence can be cloned directly into an expression vector which already contains the control sequences and an appropriate restriction site.

The expression vector may then be used to transform an appropriate host cell. A number of mammalian cell lines are known in the art and include immortalized cell lines available from the American Type Culture Collection (ATCC), such as, but not limited to, Chinese hamster ovary (CHO) cells, HeLa cells, HEK293, baby hamster kidney (BHK) cells, monkey kidney cells (COS), human hepatocellular carcinoma cells (e.g., Hep G2), Madin-Darby bovine kidney ("MDBK") cells, NOS cells derived from carcinoma cells, such as sarcoma, as well as others. Similarly, bacterial hosts such as E. coli, Bacillus subtilis, and Streptococcus spp., will find use with the present expression constructs. Yeast hosts useful in the present invention include inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe and Yarrowia lipolytica. Insect cells for use with baculovirus expression vectors include, inter alia, Aedes aegypti, Autographa californica, Bombyx mori, Drosophila melanogaster, Spodoptera frugiperda, and Trichoplusia ni. The bispecific fusion proteins may also be expressed in Trypanosomes.

Depending on the expression system and host selected, the bispecific fusion protein of the present invention is produced by growing host cells transformed by an expression vector described above under conditions whereby the bispecific fusion protein of interest is expressed. The bispecific fusion protein is then isolated from the host cells and purified. If the expression system secretes the bispecific fusion protein into growth media, the bispecific fusion protein can be purified directly from the media. If the bispecific fusion protein is not secreted, it is isolated from cell lysates. The selection of the appropriate growth conditions and recovery methods are within the skill of the art. Once purified, the amino acid sequences of the bispecific fusion protein can be determined, i.e., by repetitive cycles of Edman degradation, followed by amino acid analysis by HPLC. Other methods of amino acid sequencing are also known in the art.

The bispecific fusion proteins of the present invention can be formulated into therapeutic compositions in a variety of dosage forms such as, but not limited to, liquid solutions or suspensions, tablets, pills, powders, suppositories, polymeric microcapsules or microvesicles, liposomes, and injectable or infusible solutions. The preferred form depends upon the mode of administration and the particular cancer type targeted. The compositions also preferably include pharmaceutically acceptable vehicles, carriers or adjuvants, well known in the art, such as human serum albumin, ion exchangers, alumina, and lecithin, buffer substances such as phosphates, glycine, sorbic acid, potassium sorbate, and salts or electrolytes such as protamine sulfate. Suitable vehicles are, for example, water, saline, dextrose, glycerol, ethanol, or the like, and combinations thereof. Actual methods of preparing such compositions are known, or will be apparent, to those skilled in the art. See, e.g., Remington's Pharmaceutical Sciences, Mack Publishing Company, Easton, Pa., 18th edition, 1990.

The above compositions can be administered using conventional modes of delivery including, but not limited to, intravenous, intraperitoneal, oral, intralymphatic, or subcutaneous administration. Local administration to a tumor in question, or to a site of inflammation, e.g., direct injection into an arthritic joint, will also find use with the present invention.

Therapeutically effective doses will be easily determined by one of skill in the art and will depend on the severity and course of the disease, the patient's health and response to treatment, and the judgment of the treating physician.

### Experimental

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

### Example 1

### Production of anti-CD3/anti-PDL1 bispecific fusion protein.

The anti-CD3/anti-PDL1 bispecific fusion protein of the present invention is a ~55kDa recombinant protein as assessed by western blotting (Figure 1A). The anti-CD3/anti-PDL1 bispecific fusion protein was produced in CHO cells transfected with the anti-CD3/anti-PDL1 construct (Figure 1B). To quantify the production of anti-CD3/anti-PDL1 bispecific fusion protein by the transfectants, CHO-anti-CD3/anti-PDL1 bispecific fusion protein cells were plated at 1 x 10⁷ cells/100 ml and supernatants were slot blotted and then stained with antibodies to the His tag. Quantification was performed using Image J software as shown in Figure 1 C.

Figure 2 shows that the anti-CD3/anti-PDL1 bispecific fusion protein binds to PDL1+ human tumor cells and not to PDL1⁻ human tumor cells. Binding activity and specificity were assessed by applying the anti-CD3/anti-PDL1 bispecific fusion protein or an irrelevant recombinant protein (Troy Fc) to PDL1⁺ (C8161) and PDL1⁻ (MEL1011) human tumor cells (Figure 2A) and to CD3⁺CD4⁺ and CD3⁺CD8⁺ human T-cells (Figure 2B). Anti-CD3/anti-PDL1 bispecific fusion protein binding was detected using a fluorescently coupled antibody to the His-tag on the anti-CD3/anti-PDL1 bispecific fusion protein. Ability to simultaneously bind PDL1 and CD3 was shown by binding soluble PDL1 (sPDL1) to the anti-CD3/anti-PDL1 bispecific fusion protein to CD3⁺ T-cells as shown in Figure 2C.

### Example 2

Cytotoxicity of 2 Chronic Myelogenous Leukemia (CML) and 2 non-CML cancer cell lines using PBMC from healthy donors and from cancer patients.

To demonstrate that the anti-CD3/anti-PDL1 bispecific fusion protein of the present invention has therapeutic efficacy it was essential to demonstrate (i) the anti-CD3/anti-PDL1 bispecific fusion protein activates T-cells from healthy donors; (ii) activated T-cells kill PDL1⁺ tumor cells and do not have off-target activity; (iii) the anti-CD3/anti-PDL1 bispecific fusion protein activates T-cells that are cytotoxic for multiple PDL1⁺ human tumor cells; (iv) the anti-CD3/anti-PDL1 bispecific fusion protein activates cytotoxic T-cells from cancer patients; and (v) the anti-CD3/anti-PDL1 bispecific fusion protein has the ability to active CD4⁺, CD8⁺ and CD3⁺.
(i) The anti-CD3/anti-PDL1 bispecific fusion protein activates T-cells from healthy donors. Peripheral blood mononuclear cells (PBMC) containing T-cells from healthy human donors were mixed with the anti-CD3/anti-PDL1 bispecific fusion protein (6.5 ng/ml) or an equal amount of an irrelevant control recombinant protein in the presence or absence of PDL1⁺ human melanoma (C8161) cells (Figure 3). T-cell activation was measured by assessing T-cell production of interferon gamma (IFNγ), a key indicator of T-cell activation. The anti-CD3/anti-PDL1 bispecific fusion protein induced high levels of IFNγ in the PBMC + anti-CD3/anti-PDL1 bispecific fusion protein + tumor cell samples. Significantly lower levels of IFNγ were induced in control PBMC + anti-CD3/anti-PDL1 bispecific fusion protein samples, and essentially no IFNγ was induced in the absence of the anti-CD3/anti-PDL1 bispecific fusion protein, demonstrating that the anti-CD3/anti-PDL1 bispecific fusion protein activates T-cells from healthy donors. This experiment also demonstrated that relatively low concentrations of the anti-CD3/anti-PDL1 bispecific fusion protein (<6.5 ng/ml) are needed to activate T-cells in the presence of tumor.
(ii) The anti-CD3/anti-PDL1 bispecific fusion protein-activated T-cells kill PDL1⁺ tumor cells and do not have off-target activity. PBMC from healthy human donors were incubated at 37°C, 5% CO₂ at a 20:1, 10:1, 5:1, or 2.5:1 ratio with Cell Tracker violet-labeled melanoma (C8161) cells in the presence or absence of the anti-CD3/anti-PDL1 bispecific fusion protein (5.2 ng/ml). After 48 hrs. the cells were harvested. Half of the cells were stained with fluorescently-tagged antibodies to CD8, CD25, and PD1; the other half were incubated with the viability dye 7AAD. Flow cytometry analysis revealed that in the presence of tumor, the anti-CD3/anti-PDL1 bispecific fusion protein-activated T-cells as assessed by expression of the activation marker CD25 (alpha chain of the IL-2 receptor). Contrary to other activation strategies, the checkpoint inhibitor receptor PD1 was only minimally expressed as shown in Figure 4A. Approximately 70% of PDL1⁺ melanoma cells were killed in the presence of the anti-CD3/anti-PDL1 bispecific fusion protein at the 20:1 ratio, and killing titered out to ~55% at the 2.5:1 ratio. Background levels of killing in the absence of the anti-CD3/anti-PDL1 bispecific fusion protein or PBMC ranged from ~5%-20% (Figure 4B).

To ascertain that the anti-CD3/anti-PDL1 bispecific fusion protein cytotoxic activity is specific for PDL1⁺ target cells, PDL1- human MEL1011 cells were used as targets (Figure 5). Less than 5% of MEL1011 cells were killed vs. ~43% of PDL1⁺ C8161 cells. These results demonstrate that the anti-CD3/anti-PDL1 bispecific fusion protein in conjunction with PBMC efficiently kills PDL1⁺ tumor cells and does not have significant off-target effects on PDL1- tumor cells.

(iii) The anti-CD3/anti-PDL1 bispecific fusion protein activates T-cells that are cytotoxic for multiple PDL1⁺ human tumor cells. To determine if the anti-CD3/anti-PDL1 bispecific fusion protein is globally effective against PDL1⁺ tumors, two lines of chronic myelogenous leukemia (CML) were tested as target tumor cells. The MEG-01 and KU812F CML cell lines contain ~21% and 30% PDL1+ cells (Figure 6A). Therefore, anti-CD3/anti-PDL1 bispecific fusion protein-mediated cytotoxicity would be expected to be in the range of 20%-30%. Using the same procedure as described for Figure 4, anti-CD3/anti-PDL1 bispecific fusion protein activated T-cells, as assessed by expression of the T-cell activation markers CD25 and CD69 (Figure 6B), and killed ~25-30% of MEG-01 and KU812F cells (Figure 6C).

To further confirm that the anti-CD3/anti-PDL1 bispecific fusion protein of the present invention targets PDL1⁺ tumor cells regardless of the type of tumor, a lung adenocarcinoma line, H358, which has >99% PDL1⁺ cells was tested and exhibited >50% tumor cell death (Figure 7). These studies demonstrate that the anti-CD3/anti-PDL1 bispecific fusion protein of the present invention activates T-cells from healthy donors that are cytotoxic for PDL1⁺ CML, melanoma, and lung cancer cells.

(iv) The anti-CD3/anti-PDL1 bispecific fusion protein of the present invention activates T-cells from cancer patients that are cytotoxic for PDL1⁺ tumor cells. Since the anti-CD3/anti-PDL1 bispecific fusion protein of the present invention has the potential to be efficacious in any cancer patient with PDL1⁺ tumor cells, PBMC from a patient with small cell lung cancer (SCLC) were tested. Using the cytotoxicity assay described in Figure 4, PDL1⁺ H358 lung cancer cells and PDL1-MEL1011 cells were Cell Tracker violet labeled and incubated with PBMC + anti-CD3/anti-PDL1 bispecific fusion protein of the present invention, PBMC, or the anti-CD3/anti-PDL1 bispecific fusion protein. Approximately 38% of the PDL1⁺ C8161 cells vs. ~15% of the PDL1-MEL1011 melanoma cells were killed when incubated with PBMC plus the anti-CD3/anti-PDL1 bispecific fusion protein of the present invention as shown in Figure 8A. To determine the actual T-cell to tumor cell ratio, the cells from panel A were labeled with fluorescently-tagged antibodies to CD3, CD4 and CD8, and the percent of CD3⁺CD4⁺ and CD3⁺CD8⁺ T-cells assessed (Figure 8B). Although the PBMC to tumor ratio was 20:1, the actual CD8 to tumor ratio was 1.29:1 and the CD4 to T-cell ratio was 2.17:1. These results demonstrate that the anti-CD3/anti-PDL1 bispecific fusion protein of the present invention activates T-cells from cancer patients and that ratios less than three (3) T-cells per tumor cell are needed for killing.

(v) The anti-CD3/anti-PDL1 bispecific fusion protein of the present invention mediates tumor cell cytotoxicity by activating both CD4⁺ and CD8⁺ T-cells, and it is theorized also CD3⁺ NKT cells. Optimal T-cell-based immunotherapy should activate the maximum number of effector cells. CD8⁺ T-cells and natural killer (NK) cells are traditionally considered the optimal effector cells. Since hypothetically the anti-CD3/anti-PDL1 bispecific fusion protein of the present invention should activate any CD3⁺ cell, it has the potential to activate CD3⁺CD8⁺ and CD3⁺CD4⁺ T-cells, as well as CD3⁺NKT-cells. To determine which cells are activated and have cytotoxic activity, PBMC were depleted for CD4⁺, CD8⁺, or CD4⁺ plus CD8⁺ cells and subsequently tested for cytotoxic activity in the presence of the anti-CD3/anti-PDL1 bispecific fusion protein of the present invention and C8161 melanoma cells. Depleted populations contained <1% of the depleted cells (Figure 9A). Simultaneous depletion of CD4⁺ plus CD8⁺ T-cells reduced killing by 61% and depletion of CD4+ cells by 34% (Figure 9B). Killing was not reduced by depletion of CD8+ cells. Since depletion of one cell populations results in increased numbers of the other populations, these results indicate that both additional CD3⁺ non-CD8⁺ or -CD4⁺ cells are rendered cytotoxic by the anti-CD3/anti-PDL1 bispecific fusion protein of the present invention. Since NKT-cells have known anti-tumor cytotoxic activity and are CD3⁺, they may be another population activated by the anti-CD3/anti-PDL1 bispecific fusion protein of the present invention. The cells of panel B were further stained with fluorescently-tagged antibodies to CD3, CD4, CD8, and the marker CD107a which identifies cells containing granules releasing the lytic molecule perforin which is responsible for target cell lysis (Figure 9C). Gated CD3⁺CD4⁺ and CD3⁺CD8⁺ populations contained >45% CD107a⁺ cells. These results further confirm that the anti-CD3/anti-PDL1 bispecific fusion protein of the present invention renders both CD4⁺ and CD8⁺ T-cells cytotoxic for PDL1⁺ targets.

The anti-CD3/anti-PDL1 bispecific fusion protein of the present invention comprises the variable regions of the heavy (V_{H}) and light (V_{L}) chains of antibodies that bind to the CD3 component of the T-cell antigen receptor and to PDL1 as shown in Figure 10. This novel recombinant protein allows for activation of T-cells through CD3, while simultaneously redirecting T-cells to PDL1⁺ tumor cells, neutralizing PD1-mediated suppression, and killing the tumor cells. The anti-CD3 sequences from the OKT3 clone⁵ and the anti-PDL1 sequences from the 12A4 clone⁶ were used in the fusion protein since the CD3 sequence has shown efficacy in other fusion proteins⁷ and PDL1 antibody has shown efficacy as a monotherapy^{8,9}.

To function, the anti-CD3/anti-PDL1 bispecific fusion protein must bind to CD3 on T-cells and PDL1 on tumor cells. Binding activity was confirmed by comparing the binding of a fluorescently tagged anti-BiTE antibody by PDL1⁺ human melanoma and CD3⁺ human T-cells either incubated with or without the anti-CD3/anti-PDL1 bispecific fusion protein.

Figure 11 shows that in contrast to the OKT3 antibody, the anti-CD3/anti-PDL1 bispecific fusion protein does not induce PDL1 expression on activated T-cells. PBMC from healthy human donors were incubated with PDL1+ human melanoma cells (C8161) in the presence or absence of the anti-CD3/anti-PDL1 bispecific fusion protein (0.12pg/mL) or with OKT3 (anti-CD3) mAb (10pg/mL). At the end of the 48 hr incubation, cells were harvested and stained for CD4, CD8, and PDL1. Gated CD4+ and CD8+ cells were analyzed for PDL1.

Although the invention has been described with reference to the above examples, it will be understood that modifications and variations are encompassed within the spirit and scope of the invention. Accordingly, the invention is limited only by the following claims.

### References

(1) Brahmer, J R, et al., Safety and activity of anti-PDL1 antibody in patients with advanced cancer. N Engl J Med 366, 2455-2465 (2012).
(2) Topalian, S L, et al., Safety, activity, and immune correlates of anti-PD-1 antibody in cancer. N Engl J Med 366, 2443-2454 (2012).
(3) Topalian, S L, et al., Survival, durable tumor remission, and long-term safety in patients with advanced melanoma receiving nivolumab. J Clin Oncol 32, 1020-1030 (2014).
(4) Ostrand-Rosenberg, S, L A Horn & S T Haile. The Programmed Death-1 Immune-Suppressive Pathway: Barrier to Antitumor Immunity. J Immunol 193, 3835-3841 (2014).
(5) Woodle, E S, et al., Humanized OKT3 antibodies: successful transfer of immune modulating properties and idiotype expression. J Immunol 148, 2756-2763 (1992).
(6) Korman, A, et al., Human monoclonal antibodies to programmed death ligand 1 (PDL1). U.S. Pat. 20130122014 A1 (2013) (2013).
(7) Baeuerle, P A & C Reinhardt. Bispecific T-cell engaging antibodies for cancer therapy. Cancer Res 69, 4941-4944 (2009).
(8) Brahmer, J R. Immune checkpoint blockade: the hope for immunotherapy as a treatment of lung cancer? Semin Oncol 41, 126-132 (2014).
(9) Drake, C G, et al., Breathing new life into immunotherapy: review of melanoma, lung and kidney cancer. Nat Rev Clin Oncol 11, 24-37 (2014).

## Claims

1. An anti-CD3/anti-PDL1 bispecific fusion protein comprising a variable region of a heavy chain of an anti-CD3 antibody, a variable region of a light chain of an anti-CD3 antibody and an amino acid linker positioned therebetween and attached to each anti-CD3 variable region and that binds to a CD3 receptor on a T-cell; and
a variable region of a heavy chain of an anti PDL1 antibody, a variable region of a light chain of an anti PDL1 antibody and an amino acid linker positioned therebetween and attached to each anti-PDL1 variable region that binds to a Programmed Death Ligand 1 (PDL1) on a tumor cell to activate T-cells through CD3 and simultaneously redirecting T-cells to PDL1+ tumor cells, neutralizing PD1-mediated suppression, and killing the tumor cells, wherein the variable region of the heavy chain of the anti-PDL1 antibody is linked through an amino acid linker to the variable region of the heavy chain of the anti-CD3 antibody wherein the amino acid linker is sufficient number of amino acid residues for binding of the anti-CD3/anti-PDL1 bispecific fusion protein to individual targets, wherein the variable region of the light chain of the PDL1 antibody and the variable region of the heavy chain of the anti-PDL1 antibody consists of amino acid residues SEQ ID NOs: 5 and 6 respectively and the wherein the variable region of the light chain of the anti-CD3 antibody and the variable region of the heavy chain of the anti-CD3 antibody consists of amino acid residues SEQ ID NOs: 7 and 8 respectively.

2. A composition comprising the anti-CD3/anti-PDL1 bispecific fusion protein according to claim 1.

3. The composition according to claim 2, further comprising an anticancer therapy selected from the group consisting of a chemotherapeutic molecule, small molecule kinase inhibitor, hormonal agent and cytotoxic agent.

4. An in vitro method of preparing an anti-CD3/anti-PDL1 bispecific fusion protein according to claim 1, the method comprising:
transfecting a host cell with polynucleotide sequences that encode the variable regions of the heavy and light chains of the anti-CD3/anti-PDL1 bispecific fusion protein to produce a transformed host cell, wherein the polynucleotide sequences encode the variable regions of the light and heavy chains of the anti-CD3 antibody and the variable region of the light and heavy chains of the anti-PDL1 antibody and linkers positioned between the variable regions and having a nucleotide sequence of SEQ ID NO: 9; and
maintaining the transformed host cell under biological conditions sufficient for expression of the bispecific fusion protein having an amino acid sequence of SEQ ID NO: 10.

5. The anti-CD3/anti-PDL1 bispecific fusion protein according to claim 1, wherein the amino acid linkers comprise at least one sequence of amino acid residues selected from SEQ ID NOs. 12, 14 or 16.

## Patentansprüche

1. Ein anti-CD3/anti-PDL1 bispezifisches Fusionsprotein, umfassend einen variablen Bereich einer schweren Kette eines Anti-CD3-Antikörpers, einen variablen Bereich einer leichten Kette eines Anti-CD3-Antikörpers und einen dazwischen positionierten Aminosäurelinker, der an jede anti-CD3-variable Region gebunden ist, und an einen CD3-Rezeptor auf einer T-Zelle bindet; und
ein variabler Bereich einer schweren Kette eines Anti-PDL1-Antikörpers, ein variabler Bereich einer leichten Kette eines Anti-PDL1-Antikörpers und einen dazwischen positionierten Aminosäurelinker, der an jede anti-PDL1-variable Region gebunden ist, die an einen Programmed Death Ligand 1 (PDL1) auf einer Tumorzelle bindet, um T-Zellen durch CD3 zu aktivieren und gleichzeitig T-Zellen zu PDL1+-Tumorzellen umzuleiten, die PD1-vermittelte Suppression zu neutralisieren und die Tumorzellen zu töten, wobei die variable Region der schweren Kette des Anti-PDL1-Antikörpers durch einen Aminosäurelinker mit der variablen Region der schweren Kette des Anti-CD3-Antikörpers verbunden ist, wobei der Aminosäurelinker eine ausreichende Anzahl von Aminosäureresten für die Bindung des anti-CD3/anti-PDL1 bispezifischen Fusionsproteins an einzelne Ziele aufweist, wobei der variable Bereich der leichten Kette des PDL1-Antikörpers und der variable Bereich der schweren Kette des Anti-PDL1-Antikörpers aus Aminosäureresten entsprechend SEQ ID NOs: 5 bzw. 6 bestehen und wobei der variable Bereich der leichten Kette des Anti-CD3-Antikörpers und der variable Bereich der schweren Kette des Anti-CD3-Antikörpers aus Aminosäureresten entsprechend SEQ ID NOs: 7 bzw. 8 bestehen.

2. Eine Zusammensetzung, die das anti-CD3/anti-PDL1 bispezifische Fusionsprotein nach Anspruch 1 umfasst.

3. Die Zusammensetzung nach Anspruch 2, weiterhin umfassend eine Antikrebstherapie ausgewählt aus der Gruppe bestehend aus einem chemotherapeutischen Molekül, einem kleinen Molekül Kinaseinhibitor, einem hormonellen Wirkstoff und einem zytotoxischen Wirkstoff.

4. Eine in-vitro Methode zur Herstellung eines anti-CD3/anti-PDL1 bispezifischen Fusionsproteins nach Anspruch 1,wobei die Methode umfasst:
Transfizieren einer Wirtszelle mit Polynukleotidsequenzen, die die variablen Regionen der schweren und leichten Ketten des anti-CD3/anti-PDL1 bispezifischen Fusionsproteins codieren, um eine transformierte Wirtszelle zu erzeugen, wobei die Polynukleotidsequenzen die variablen Regionen der leichten und schweren Ketten des Anti-CD3-Antikörpers sowie den variablen Bereich der leichten und schweren Ketten des Anti-PDL1-Antikörpers sowie Linker kodieren, die zwischen den variablen Regionen positioniert sind und eine Nukleotidsequenz entsprechend SEQ ID NO: 9 haben; und
Erhalten der transformierten Wirtszelle unter biologischen Bedingungen, die für die Expression des bispezifischen Fusionsproteins mit einer Aminosäuresequenz nach SEQ ID NO: 10 ausreichen.

5. Das anti-CD3/anti-PDL1 bispezifische Fusionsprotein nach Anspruch 1, wobei die Aminosäure-Linker mindestens eine Sequenz von Aminosäureresten aus den SEQ-ID-NOs 12, 14 oder 16 umfasst.

## Revendications

1. Protéine de fusion bispécifique anti-CD3/anti-PDL1 comprenant une région variable d'une chaîne lourde d'un anticorps anti-CD3, une région variable d'une chaîne légère d'un anticorps anti-CD3 et un lieur d'acides aminés positionné entre celles-ci et attaché à chaque région variable anti-CD3 et qui se lie à un récepteur CD3 sur une cellule T, et
une région variable d'une chaîne lourde d'un anticorps anti-PDL1, une région variable d'une chaîne légère d'un anticorps anti-PDL1 et un lieur d'acides aminés positionné entre celles-ci et attaché à chaque région variable anti-PDL1 qui se lie à un ligand de mort cellulaire programmée 1 (PDL1) sur une cellule tumorale pour activer des cellules T par l'intermédiaire de CD3 et rediriger simultanément les cellules T vers des cellules tumorales PDL1+, neutraliser la suppression médiée par le PDL et tuer les cellules tumorales, dans laquelle la région variable de la chaîne lourde de l'anticorps anti-PDL1 est liée, par l'intermédiaire d'un lieur d'acides aminés, à la région variable de la chaîne lourde de l'anticorps anti-CD3, dans laquelle le lieur d'acides aminés est un nombre de résidus d'acides aminés suffisant pour la liaison de la protéine de fusion bispécifique anti-CD3/anti-PDL1 à des cibles individuelles, dans laquelle la région variable de la chaîne légère de l'anticorps PDL1 et la région variable de la chaîne lourde de l'anticorps anti-PDL1 consistent en des résidus d'acides aminés des SÉQ ID N° : 5 et 6 respectivement, et dans laquelle la région variable de la chaîne légère de l'anticorps anti-CD3 et la région variable de la chaîne lourde de l'anticorps anti-CD3 consistent en des résidus d'acides aminés des SÉQ ID N° : 7 et 8 respectivement.

2. Composition comprenant la protéine de fusion bispécifique anti-CD3/anti-PDL1 selon la revendication 1.

3. Composition selon la revendication 2, comprenant en outre une thérapie anticancéreuse choisie parmi le groupe constitué d'une molécule chimiothérapeutique, d'un inhibiteur de kinase à petites molécules, d'un agent hormonal et d'un agent cytotoxique.

4. Procédé in vitro de préparation d'une protéine de fusion bispécifique anti-CD3/anti-PDL1 selon la revendication 1, le procédé comprenant de :
transfecter une cellule hôte avec des séquences de polynucléotides qui codent pour les régions variables des chaînes lourdes et légères de la protéine de fusion bispécifique anti-CD3/anti-PDL1 pour produire une cellule hôte transformée, dans lequel les séquences de polynucléotides codent pour les régions variables des chaînes légères et lourdes de l'anticorps anti-CD3 et la région variable des chaînes légères et lourdes de l'anticorps anti-PDL1 et des lieurs positionnés entre les régions variables et ayant une séquence de nucléotides de la SÉQ ID N° : 9, et
maintenir la cellule hôte transformée dans des conditions biologiques suffisantes pour l'expression de la protéine de fusion bispécifique ayant une séquence d'acides aminés de la SÉQ **ID** N° : 10.

5. Protéine de fusion bispécifique anti-CD3/anti-PDL1 selon la revendication 1, dans laquelle les lieurs d'acides aminés comportent au moins une séquence de résidus d'acides aminés choisis parmi les SÉQ ID N° 12, 14 ou 16.
